# FASCICULE DE BREVET EUROPEEN

(11) **EP 0 233 432 B1**
(45) Date de publication et mention de la délivrance du brevet: **15.03.1989**
(21) Numéro de dépôt: 86402939.2
(22) Date de dépôt: 24.12.1986
(51) Int. Cl.: C07C 149/18, C07F 9/54, C07C 149/20, C07D 213/20, C07C 149/267

(54) **Composés polyfluoroalkylthio-méthyliques, leurs procédés de préparation et leurs applications comme agents tensio-actifs ou précurseurs de ces derniers**
Polyfluoralkylthiomethylverbindungen, Verfahren zu ihrer Herstellung und ihre Verwendung als tensioaktive Verbindungen oder als Vorläufer dazu
Polyfluoralkylthiomethyl compounds, processes for their preparation and their use as tensio-active compounds or as precursors therefor

(30) Priorité: 07.01.1986 FR 8600135
(43) Date de publication de la demande: 26.08.1987
(73) Titulaire: ELF ATOCHEM S.A., 92800 Puteaux (FR)
(72) Inventeur: Marty, Frédérique, F-06000 Nice (FR); Rouvier, Emile, F-06690 Tourrette-le-Vens (FR); Cambon, Aimé, F-06000 Nice (FR)
(74) Mandataire: Leboulenger, Jean

(56) Documents cités:
- FR-A- 2 516 920
- US-A- 3 172 910

## Description

La présente invention concerne de nouveaux composés polyfluorés et, plus particulièrement, des alcools polyfluorés du type hémithioacétal qui constituent de précieux intermédiaires pour la synthèse de dérivés polyfluorés, notamment d'agents tensio-actifs ioniques.

Dans le brevet US-A 3 655 732 relatif, entre autres, à des monomères acryliques de formule générale: 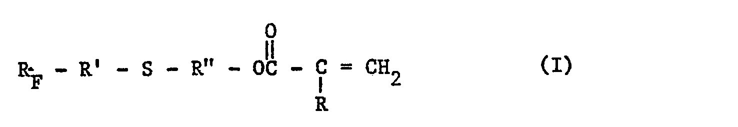 dans laquelle R est un atome d'hydrogène ou un radical méthyle, R_{F} désigne un radical perfluoroalkyle et R' et R" représentent des ponts alkylène, il est indiqué (colonne 4, lignes 63-72) que les hémithioacétals de formule:
R_{F}-R'-S-CH₂0H (II)

ne sont pas accessibles par les méthodes connues. Les monomères acryliques correspondants: 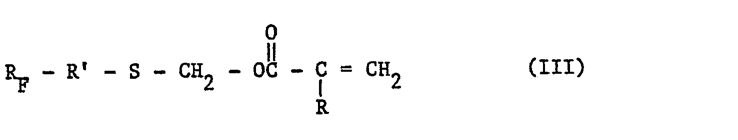 ne pouvant donc pas être obtenus par estérification directe, il est suggéré dans le brevet précité de préparer indirectement ces monomères en faisant réagir un thiol R_{F}-R'-SH avec le formaldéhyde et l'acide chlorhydrique pour former un dérivé chlorométhylé R_{F}-R'-S-CH₂CI qu'on fait réagir ensuite avec l'acrylate ou le méthacrylate de sodium; les conditions opératoires ne sont pas indiquées. D'autre part, d'après la littérature (H. Bôhme, Chem. Ber. 69,1610; brevet DE 845 511; H. Bohme et al., Ann. 563. 54), la réaction d'un thiol avec le formaldéhyde et l'acide chlorhydrique doit être effectuée à très basse température (de -15 à -5_{°}C). Il a cependant été constaté qu'appliquée aux thiols fluorés R_{F}-R'-SH, cette réaction ne conduit pas aux dérivés chlorométhylés, mais aux thioacétals (R_{F}-R'-S)₂CH₂.

Il a maintenant été trouvé que les hémithioacétals (II) peuvent être obtenus par action du trioxyméthylène (ou s-trioxane) sur un polyfluoroalcane-thiol. En outre, à partir de ces nouveaux alcools polyfluorés, on a pu préparer divers dérivés utilisables comme agents tensio-actifs ou précurseurs de ces derniers.

D'une façon générale, l'invention a donc pour objet les composés polyfluoroalkylthio-méthyliques de formule générale:
R_{F}(CH₂)ₘ-S-CH₂-A (IV)

dans laquelle R_{F} représente un radical perfluoroalkyle, linéaire ou ramifié, contenant de 2 à 16 atomes de carbone, m est un nombre entier allant de 1 à 4, de préférence égal à 2, et A représente un groupe hydroxyle, un atome de chlore ou de brome, un groupement ammonium dérivé d'un amine secondaire ou tertiaire, un groupement phosphonium ou un groupement ionique ou non ionique -Q-(CH₂)ₙ-Z, Q représentant un atome d'oxygène ou de soufre, n le nombre 0, 1 ou 2 et Z un radical alkyle, aryle ou alkylaryle éventuellement substitué.

Parmi ces composés, on préfère ceux dans lesquels le radical perfluoroalkyle R_{F} est linéaire et contient 4, 6, 8, ou 10 atomes de carbone.

Quand A représente un groupement ammonium, celui-ci est de préférence choisi par ceux de formules: 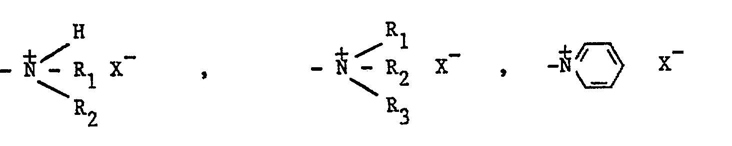 et 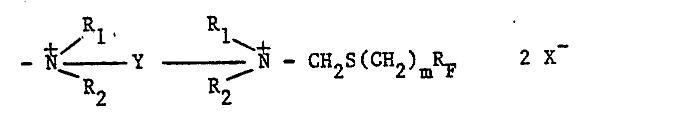 dans lesquelles les symboles R₁, R₂ et R₃ peuvent être identiques ou différents et représentent chacun un radical alkyle, phényle ou benzyle éventuellement substitué, Y désigne un pont alkylène de 2 à 6 atomes de carbone éventuellement interrompu par un atome d'oxygène, et X- représente un anion monovalent ou son équivalent. Le radical alkyle peut être linéaire ou ramifié et contenir de 1 à 18 atomes de carbone. Comme substituants éventuellement présents sur les radicaux alkyle, phényle et benzyle, on peut mentionner les atomes d'halogène, les groupes hydroxyle et nitrile, et les fonctions ester, acide, sulfonate, sulfate ou carboxylate.

Le groupement phosphonium que peut représenter A est de préférence un groupement de formule: 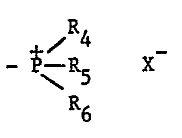 dans laquelle les symboles R₄, R₅ et R₆ peuvent être identiques ou différents et représentent chacun un radical alkyle ou aryle (notamment phényle), X- ayant la même signification que précédemment. Les radicaux alkyle peuvent contenir de 1 à 6 atomes de carbone.

Quand A représente un groupement -Q-(CH₂)ₙ-Z, le radical alkyle, aryle ou alkylaryle Z peut porter des substituants hydrophiles, ioniques ou non ioniques, tels que des groupes hydroxyle, carbalcoxy (par exemple, carbométhoxy ou carbéthoxy), aryloxycarbonyle (par exemple phénoxycarbonyle), ammonium, carboxylate, bétaïne, sulfonate, sulphate ou phosphate, et/ou des substituants non hydrophiles tels que des atomes d'halogène ou des groupes nitrile, acyle (par exemple acétyle ou benzoyle) ou dialkylamino (alkyl en C₁-C₄).

Pour préparer les composés de formule (IV) dans lesquels A est un groupe hydroxyle, c'est-à-dire les alcools de formule:

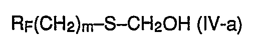

on fait réagir le trioxyméthylène sur un polyfluoroalcane-thiol RF(CH₂)ₘ-SH, à raison de trois moles de thiol pour une mole de trioxyméthylène. La réaction est effectuée en présence d'une amine tertiaire comme catalyseur, à raison de 2.10-⁴ à 2.10-1 mole par mole de trioxyméthylène. Cette amine tertiaire est de préférence la triéthylamine, mais on peut également utiliser d'autres amines comme, par exemple, la tripropylamine, la tributylamine, la diméthyléthylamine, la diméthylcyclohexylamine, la diméthylaniline, la N-méthylpipéridine et la pyridine, ainsi que des hydroxydes d'ammonium quaternaire tels que l'hydroxyde de triméthyl benzyl ammonium (Triton B). Les polyfluoroalcane-thiols, utilisés comme produits de départ, sont des composés bien connus (voir par exemple les brevets US 2 894 991, US 3 088 849, US 3 544 663, FR 1 221 415 et FR 2 083 422).

La réaction peut être effectuée à une température allant jusqu'à 50_{°}C, éventuellement au sein d'un solvant organique inerte (par exemple, un éther ou un hydrocarbure halogéné). Cependant, pour éviter la formation concomitante du disulfure [R_{F}(CH₂)ₘS]₂ qui, bien qu'en faible proportion, est difficilement séparable, il est particulièrement souhaitable d'opérer à environ 0_{°}C sous atmosphère d'un gaz inerte (notamment l'azote) et en l'absence de solvant. Dans ces conditions, on obtient un alcool (IV-a) suffisamment pur pour être utilisé tel quel.

Afin de synthétiser les autres composés de formule (IV) selon l'invention, on soumet ensuite les alcools (IV-a) à une halogénation pour former les chlorures et, de préférence, les bromures correspondants de formule:

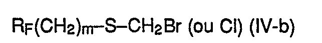

Comme agents d'halogénation, on peut utiliser, par exemple, le trichlorure ou tribromure de phosphore, le pentachlorure de phosphore, l'oxychlorure de phosphore ou le chlorure de thionyle. La réaction d'halogénation peut être effectuée à une température allant jusqu'à 80_{°}C au sein d'un solvant organique inerte. Pour éviter la formation concomitante du thioacétal [R_{F}(CH₂)ₘCH₂S]₂CH₂, on opère de préférence au voisinage de 0_{°}C sous atmosphère de gaz inerte et au sein d'un éther anhydre.

Les composés de formule (IV) dans laquelle A est un groupement ammonium peuvent être préparés par réaction d'un sulfure a-halogéné (IV-b), de préférence a-bromé, avec une amine secondaire ou tertiaire.

La réaction est de préférence réalisée en dissolvant des quantités sensiblement équimoléculaires du sulfure a-halogéné (IV-b) et de l'amine choisie dans deux ou trois volumes d'éther. Après une courte période d'induction, il se produit une réaction exothermique avec formation d'un halogénohydrate (cas des amines secondaires) ou d'un halogénure d'ammonium quaternaire (cas des amines tertiaires). Ces sels qui précipitent dans le milieu réactionnel peuvent ensuite être recristallisés, par exemple dans un mélange d'acétate d'éthyle et de chloroforme ou dans l'acétone. La plupart ont une structure cristalline et ne sont pas hygroscopiques; ils sont tous parfaitement solubles dans l'eau et conduisent à des solutions neutres.

Comme exemples non limitatifs d'amines utilisables, on peut citer plus particulièrement la triméthylami- ne, la triéthylamine, la tripropylamine, la tri-isopropyl-amine, la tributylamine, la diméthyl-éthylamine, la N,N-diméthyl-aniline, la N,N-diméthyl-benzylamine, les amines grasses N,N-diméthylées, c'est-à-dire les N,N-diméthyl-alkylamines dont le radical alkyle contient de 8 à 18 atomes de carbone (notamment la N,N-diméthyl-octylamine), la pyridine, la N,N-diméthyl-éthanolamine, la N,N-diéthyl-éthanolamine, la N,N-dibutyl-éthanolamine, la N-méthyl-diéthanolamine, la triéthanolamine, le diméthylamino-3 propionate d'éthyle, le méthylamino-benzoate d'éthyle. On peut aussi utiliser des diamines à condition d'employer une double proportion molaire de sulfure halogéné (IV-b); un exemple typique de diamine utilisable est la N,N,N',N'-tét_{F}améthyl éthylènediamine qui conduit à des sels doubles de formule:

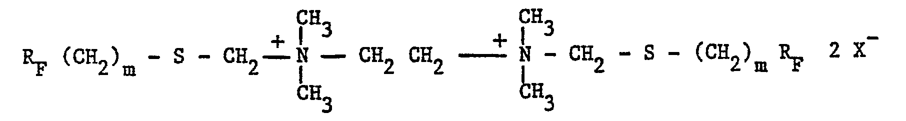

D'autres exemples de diamines sont la N,N,N',N'-tétraméthyl hexanediamine-1,6 et le N,N,N',N'-tétra- méthyl diaminodiéthyléther.

Les composés de formule (IV) dans laquelle A est un groupement phosphonium peuvent être préparés de la même façon que les sels d'ammonium en remplaçant l'amine secondaire ou tertiaire par une phosphine. La réaction étant moins rapide qu'avec les amines, il est souhaitable d'opérer au reflux. Comme exemples de phosphines utilisables, on peut citer à titre non limitatif la triphénylphosphine, la triméthylphosphi- ne, la triéthylphosphine et la tributylphosphine.

L'anion Br- ou CI- des sels d'ammonium ou de phosphonium peut, si on le désire, être facilement échangé contre un autre anion par des méthodes bien connues en soi. Comme exemples d'autres anions, on peut mentionner plus spécialement les anions iodure, nitrate, p.toluènesulfonate, sulfate, alkylsulfate, picrate.

Les sels d'ammonium ou de phosphonium selon l'invention sont de précieux agents tensio-actifs, utilisables comme additifs dans des domaines très variés en tant qu'agents mouillants, émulsifiants, dispersants, ou moussants.

Les composés de formule (IV) dans laquelle A est un groupement -Q-(CH₂)ₙ-Z peuvent être préparés par réaction d'un sulfure a-halogéné (IV-b) avec un alcoolate ou thiolate de sodium de formule:

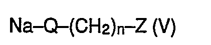

### dans laquelle les symboles Q, n et Z ont les mêmes significations que précédemment.

Cette réaction peut être effectuée à une température allant jusqu'à 80_{°}C en ajoutant le sulfure a-halogéné (IV-b) à une suspension du sel de sodium (V) dans un solvant organique inerte, par exemple un éther. Il est avantageux d'employer un excès de composé (V), cet excès pouvant aller jusqu'à 50%. Pour éviter la formation indésirable de thioacétal [R_{F}(CH₂)ₘ-S]₂CH₂, l'addition du sulfure a-halogéné (IV-b) est de préférence réalisée à 0°C et sous atmosphère inerte, la réaction pouvant ensuite être achevée à température ambiante par agitation.

Après élimination des insolubles et du solvant, les composés de formule: 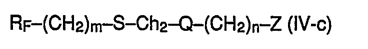 peuvent être isolés et éventuellement purifiés par des méthodes classiques telles que la distillation sous pression réduite. Lorsque Z comporte au moins un substituant hydrophile, ces composés sont utilisables comme agents tensio-actifs pour les mêmes applications que celles mentionnées précédemment à propos des sels d'ammonium ou de phosphonium. Les composés IV-c dont le radical Z n'est pas substitué ou l'est par des substituants non hydrophiles constituent de précieux intermédiaires pour la préparation d'agents tensio-actifs.

Les exemples suivants illustrent l'invention, sans la limiter.

### EXEMPLE 1

Dans un ballon de 200 ml à fond rond, muni d'un agitateur magnétique et d'une ampoule de coulée contenant 40 g (0,105 mole) de perfluorohexyl-2 éthanethiol C₆F₁₃C₂H₄SH, on introduit 3,16 g (0,035 mole) de trioxyméthylène et 10 gouttes de triéthylamine. On refroidit le ballon à l'aide d'un bain d'eau glacée et place le mélange sous atmosphère d'azote.

On procède ensuite à l'addition lente du thiol, tout en maintenant la température du mélange réactionnel à 0_{°}C jusqu'à consommation du trioxyméthylène. Le mélange réactionnel qui se présente alors sous forme d'une solution bleutée, encore légèrement opaque, est ensuite ramené à la température ambiante et maintenu sous agitation pendant 24 heures.

A la solution limpide ainsi obtenue, on ajoute de l'eau et extrait à l'éther éthylique. La phase éthérée est ensuite lavée plusieurs fois à l'eau, puis séchée sur sulfate de sodium. Après filtration et élimination de l'éther, on obtient avec un rendement de 90 à 95% le (perfluorohexyl-2 éthylthio) méthanol: C₆F₁₃C₂H₄SCH₂OH, sous forme d'un liquide incolore, à odeur caractéristique. Eb: 67-69_{°}C (6666 Pa).

En opérant de la même façon à partir des thiols: C₄FgC₂H₄SH et C₈F₁₇C₂H₄SH, on obtient respectivement les alcools suivants:

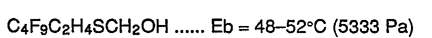

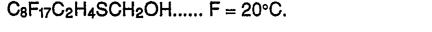

### EXEMPLE 2

Dans un ballon, surmonté d'un réfrigérant et d'une ampoule à brome, refroidi dans un bain de glace et placé sous atmosphère d'azote, on introduit 44 g (0,107 mole) de l'alcool C₆F₁₃C₂H₄SCH₂OH dans 50 ml d'éther éthylique anhydre. Par l'ampoule à brome, on ajoute alors goutte à goutte 10 ml de tribromure de phosphore (0,107 mole) en solution dans 50 ml d'éther éthylique anhydre. L'addition terminée, on retire le bain d'eau glacée et achève la réaction en maintenant sous agitation à température ambiante pendant 12 heures.

Le mélange réactionnel est ensuite versé dans l'eau glacée, puis extrait à l'éther. La phase éthérée est ensuite lavée avec une solution de soude diluée (0,05%), puis plusieurs fois à l'eau. Après séchage sur sulfate de sodium, filtration et évaporation de l'éther, une distillation sous pression réduite permet d'obtenir le bromure de perfluorohexyl-2 éthylthiométhyle C₆F₁₃C₂H₄SCH₂Br. Eb = 124_{°}C (6666 Pa). Rendement: 93%.

En opérant de la même façon à partir du (perfluorobutyl-2 éthylthio) méthanol, on obtient avec un rendement de 90% le bromure de perfluorobutyl-2 éthylthiométhyle C₄FgC₂H₄SCH₂Br qui bout à 98°C sous 5333 Pa.

### EXEMPLE 3

On opére comme à l'exemple 2, mais en remplaçant le tribromure de phosphore par une quantité équimo- laire de chlorure de thionyle et en utilisant le chloroforme comme solvant (5 ml par gramme de R_{F}C₂H₄SCH₂0H). On obtient avec un rendement de 65 à 70% les composés chlorés suivants:

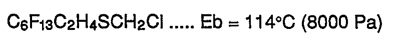

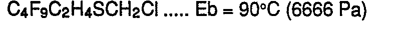

### EXEMPLE 4

A partir des amines NR₁R₂R₃ mentionnés dans la première colonne du tableau de la page 9, on a préparé les sels de formule: 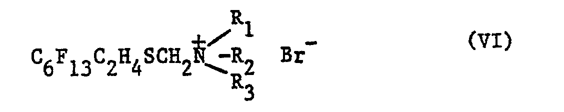 en procédant comme suit:

Dans un ballon muni d'un agitateur magnétique, on introduit des quantités équimoléculaires du sulfure a- bromé: C₆F₁₃C₂H₄SCH₂Br et d'amine NR₁R₂R₃ en solution dans deux ou trois volumes d'éther éthylique. La réaction est exothermique et pratiquement immédiate. Le sel quaternaire précipite sous la forme d'un solide blanc. L'agitation est maintenue jusqu'à consommation totale du sulfure a-bromé, puis le solide obtenu est abondamment rincé à l'éther et ensuite recristallisé par dissolution à chaud dans un mélange d'acétate d'éthyle (5 ml par gramme de solide) contenant suffisamment de chloroforme pour obtenir une solution limpide; après refroidissement et filtration, on obtient les sels quaternaires correspondants de formule (VI). Ces sels, identifiés par spectrométrie RMN, IR et de masse, sont parfaitement solubles dans l'eau. Leur point de fusion et la tension superficielle à 25_{°}C d'une solution aqueuse à 0,1% sont indiqués dans les troisième et quatrième colonnes du tableau de la page 9, la seconde colonne indiquant le rendement.

### EXEMPLE 5

On opère comme à l'exemple 4 en utilisant comme amine la N,N,N',N'-tétraméthyl éthylènediamine avec un rapport molaire:
sulfure/amine égal à 2. On obtient ainsi avec un rendement de 81 % le sel quaternaire double de formule: 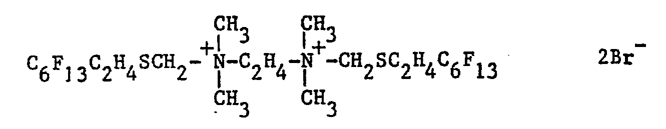 qui fond à 164-165_{°}C. La tension superficielle d'une solution aqueuse à 0,1% de ce sel est de 14,9 mN/m à 25_{°}C.

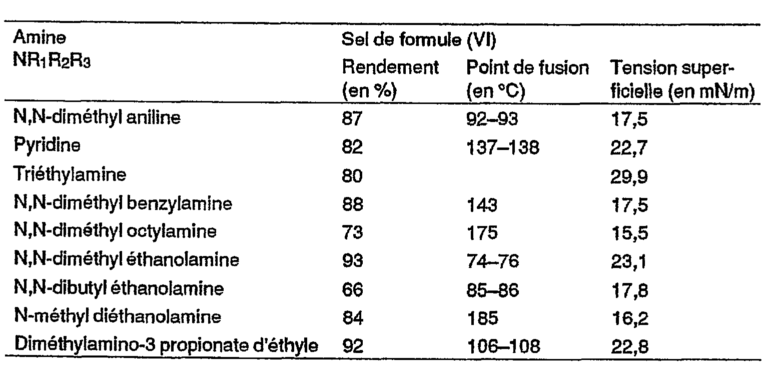

### EXEMPLE 6

Dans un ballon de 50 ml muni d'un réfrigérant, on introduit 2 g (5,36 mM) du bromure C₄F₉C₂H₄SCH₂Br et 1,82 g (6,97 mM) de triphénylphosphine en solution dans 20 ml d'éther éthylique anhydre, puis on chauffe au reflux sous agitation pendant 48 heures. Il se forme un précipité blanc qui est filtré sur verre fritté, rincé abondamment à l'éther, puis recristallisé dans l'acétate d'éthyle.

On obtient ainsi le sel de formule:
C₄F₉C₂H₄SCH₂+P(C₆H₅)₃ Br
identifié par spectrométrie RMN, IR et par analyse élémentaire.
EXEMPLE 7

Dans un ballon surmonté d'un réfrigérant et d'une ampoule à brome, refroidi dans un bain de glace et placé sous atmosphère inerte, on introduit 1,09 g (8,24 mmoles) de thiophénate de sodium en suspension dans 30 ml d'éther éthylique anhydre. Par l'ampoule à brome, on ajoute goutte à goutte une solution de 3 g (6,34 mmoles) de CsF_{I}aC₂H4SCH₂Br dans 20 ml d'éther.

A la fin de l'addition, le bain de glace est retiré et la réaction est achevée en maintenant sous agitation à température ambiante pendant 24 heures. Le mélange réactionnel est ensuite filtré sur verre fritté et le résidu (thiophénate de sodium en excès et bromure de sodium formé in situ) est rincé à l'éther. Le filtrat ayant été recueilli, on élimine l'éther par évaporation et distille ensuite sous pression réduite.

On obtient ainsi avec un rendement voisin de 100% le composé C₆F₁₃C₂H₄SCH₂SC₆H₅ qui bout à 162_{°}C sous 5333 Pa et a été identifié par spectrométrie RMN, IR et de masse.

En remplaçant le thiophénate de sodium par le sel de sodium du benzylmercaptan ou du thioglycolate de méthyle, on obtient respectivement les composés suivants:

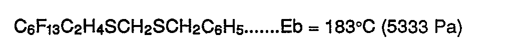

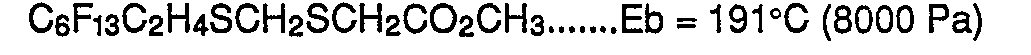

## Revendications

1. Composés polyfluoroalkylthio-méthyliques de formule générale: 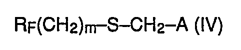 dans laquelle R_{F} représente un radical perfluoroalkyle, linéaire ou ramifié, contenant de 2 à 16 atomes de carbone, m est un nombre entier allant de 1 à 4, et A représente un groupe hydroxyle, un atome de chlore ou de brome, un groupe ammonium dérivé d'une amine secondaire ou tertaire, un groupement phosphonium ou un groupement ionique ou non ionique -Q-(CH₂)n-Z , Q représentant un atome d'oxygène ou de soufre, n le nombre 0, 1 ou 2 et Z un radical alkyle, aryle ou alkylaryle éventuellement substitué.

2. Composés selon la revendication 1, dans lesquels m est égal à 2.

3. Composés selon la revendication 1 ou 2, dans lesquels A est un groupement ammonium choisi par ceux de formules: 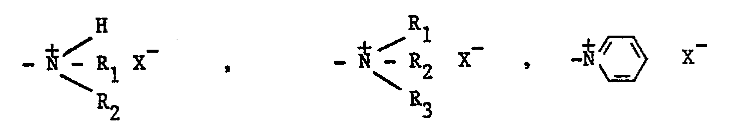 et 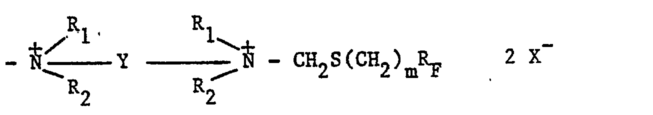 dans lesquelles les symboles Ri, R₂ et R₃, identiques ou différents, représentent chacun un radical alkyle, phényle ou benzyle éventuellement substitué, Y représente un pont alkylène de 2 à 6 atomes de carbone éventuellement interrompu par un atome d'oxygène, et X- représente un anion monovalent ou son équivalent.

4. Composés selons la revendication 3, dans lesquels l'un au moins des radicaux Ri, R₂ et R₃ est substitué par un atome d'halogène, un groupe hydroxyle ou nitrile, ou une fonction ester, acide, sulfonate, sulfate ou carboxylate.

5. Composés selon la revendication 1 ou 2, dans lesquels A est un groupement phosphonium: 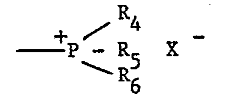 où les symboles R₄, Rₛ et Rₛ, identiques ou différents, représentent chacun un radical alkyle ou aryle, et X- représente un anion monovalent ou son équivalent.

6. Composés selon la revendication 1 ou 2, dans lesquels A est un groupement -Q-(CH₂)n-Z dont le radical alkyle, aryle ou alkylaryle Z porte au moins un substituant hydrophile, ionique ou non ionique.

7. Composés selon la revendication 1 ou 2, dans lesquels A est un groupement -Q-(CH₂)ₙ-Z dont le radical alkyle, aryle ou alkylaryle Z n'est pas substitué ou l'est par un ou plusieurs substituants non hydrophiles.

8. Procédé de préparation d'un alcool de formule: 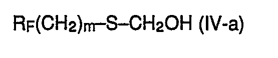 selon la revendication 1, caractérisé en ce que l'on fait réagir le trioxyméthylène sur un polyfluoroalcane-thiol R_{F}(CH₂)ₘ-8H, à raison de trois moles de thiol pour une mole de trioxyméthylène.

9. Procédé selon la revendication 8, dans lequel on opère à environ 0_{°}C sous atmosphère de gaz inerte et en l'absence de solvant.

10. Procédé de préparation d'un halogènure polyfluoroalkylthio-méthylique de formule: 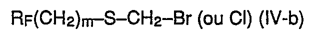 selon la revendication 1, caractérisé en ce que l'on fait réagir un agent d'halogénation sur l'alcool correspondant de formule:

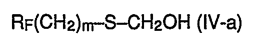

11. Procédé selon la revendication 10, dans lequel on opère à environ 0_{°}C sous atmosphère de gaz inerte et au sein d'un éther anhydre.

12. Procédé de préparation des composés selon la revendication 1 dans lesquels A est un groupement ammonium, phosphonium ou -Q-(CH₂)n-Z, caractérisé en ce que l'on fait réagir un halogénure polyfluoroalkylthio-méthylique de formule: 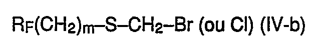 avec respectivement une amine secondaire ou tertiaire, une phosphine ou un alcoolate ou thiolate de formule: 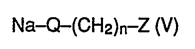 les symboles R_{F}, Q, m, n et Z ayant les mêmes significations que dans la revendication 1.

13. Utilisation des composés selon l'une des revendications 3 à 6 comme agents tensio-actifs.

14. Utilisation des composés selon la revendication 7 comme intermédiaires pour la synthèse d'agents tensio-actifs.

## Patentansprüche

1. Polyfluoralkylthiomethylverbindungen der allgemeinen Formel: 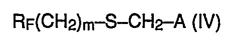 in der R_{F} einen Perfluoralkylrest darstellt, der linear oder verzweigt ist und 2 bis 16 Kohlenstoffatome enthält, m eine ganze Zahl von 1 bis 4 ist und A eine Hydroxylgruppe darstellt, ein Chlor- oder Bromatom, eine Ammoniumgruppe abgeleitet von einem sekundären oder tertiären Amin, eine Phosphoniumgruppe oder eine ionische oder nichtionische Gruppe -Q-(CH₂)ₙ-Z, in der Q ein Sauerstoffatom oder Schwefelatom darstellt, n die Zahl 0, 1 oder 2 ist und Z einen Alkyl-, Aryl- oder Alkylarylrest darstellt, der gegebenenfalls substituiert ist.

2. Verbindungen nach Anspruch 1, bei denen m gleich 2 ist.

3. Verbindungen nach Anspruch 1 oder 2, bei denen A ein Ammoniumrest ausgewählt aus denjenigen der Formeln: 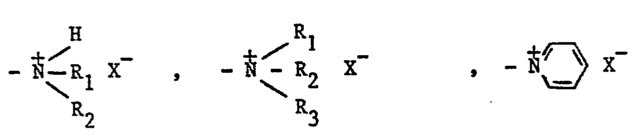 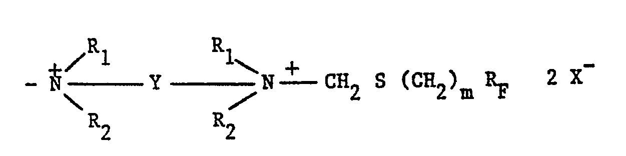 ist, in denen die Reste R₁, R₂ und R₃ gleich oder verschieden sind und jeweils einen Alkylrest, Phenyl-oder Benzylrest darstellen, der gegebenenfalls substituiert ist, Y eine Alkylenbrücke mit 2 bis 6 Kohlenstoffatomen darstellt, die gegebenenfalls von einem Sauerstoffatom unterbrochen ist, und X- ein monovalentes Anion oder dessen Aquivalent darstellt.

4. Verbindungen nach Anspruch 3, in denen wenigstens einer der Reste Ri, R₂ und R₃ durch ein Halogenatom, eine Hydroxyl- oder Nitrilgruppe oder eine Ester-, Säure-, Sulfonat-, Sulfat- oder Carboxylatfunktion substituiert ist.

5. Verbindungen nach Anspruch 1 oder 2, bei denen A eine Phosphoniumgruppe: 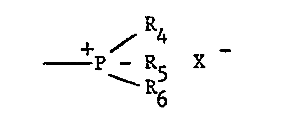 ist, in der die Reste R₄, Rₛ und R₆ gleich oder verschieden sind und jeweils einen Alkyl- oder Arylrest darstellen, und in der X- ein monovalentes Anion oder dessen Äquivalent darstellt.

6. Verbindungen nach Anspruch 1 oder 2, bei denen A eine Gruppierung -Q-(CH₂)ₙ-Z ist, in der der Alkylrest, Arylrest oder Alkylarylrest Z wenigstens einen hydrophilen, ionischen oder nichtionischen Substituenten trägt.

7. Verbindungen nach Anspruch 1 oder 2, bei denen A eine Gruppierung -Q-(CH₂)ₙ-Z ist, in der der Alkyl-, Aryl- oder Alkylarylrest Z nicht substituiert ist oder mit einem oder mehreren nicht hydrophilen Substituenten substituiert ist.

8. Verfahren zur Herstellung eines Alkohols der Formel 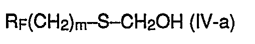 gemäß Anspruch 1, dadurch gekennzeichnet, daß man Trioxymethylen mit einem Polyfluoralkanthiol R_{F}(CH₂)m-8H im Verhältnis von drei Molen Thiol auf ein Mol Trioxymethylen reagieren läßt.

9. Verfahren nach Anspruch 8, bei dem man bei ungefähr 0_{°}C unter einer Inertgasatmosphäre und in Abwesenheit eines Solvens arbeitet.

10. Verfahren zur Herstellung eines Polyfluoralkylthiomethylhalogenids der Formel: 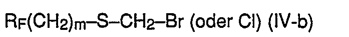 gemäß Anspruch 1, dadurch gekennzeichnet, daß man ein Halogenierungsagens mit einem Alkohol gemäß der Formel: 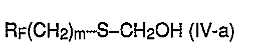 umsetzt.

11. Verfahren nach Anspruch 10, bei dem man bei ungefähr 0_{°}C unter einer Inertgasatmosphäre und in Lösung eines wasserfreien Ethers arbeitet.

12. Verfahren zur Herstellung von Verbindungen gemäß Anspruch 1, bei denen A einen Ammoniumrest, Phosphoniumrest oder einen Rest -Q-(CH₂)ₙ-Z darstellt, dadurch gekennzeichnet, daß man ein Polyfluoralkylthiomethylhalogenid der Formel: 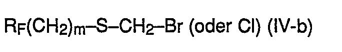 mit jeweils einem sekundären oder tertiären Amin, einem Phosphin oder einem Alkoholat oder Thiolat der Formel: 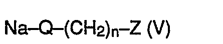 reagieren läßt, wobei die Symbole R_{F}, Q, m, n und Z die gleiche Bedeutung wie in Anspruch 1 haben.

13. Verwendung der Verbindungen gemäß einem der Ansprüche 3 bis 6 als oberflächenaktive Agenzien.

14. Verwendung der Verbindungen gemäß Anspruch 7 als Zwischenprodukte für die Synthese von oberflächenaktiven Agenzien.

## Claims

1. Polyfluoroalkylthiomethyl compounds of general formula: 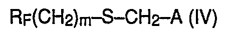 in which R_{F} denotes a straight-chain or branched perfluoroalkyl radical containing from 2 to 16 carbon atoms, m is an integer ranging from 1 to 4, and A denotes a hydroxyl group, a chlorine or bromine atom, an ammonium group derived from a secondary or tertiary amine, a phosphonium group or an ionic or nonionic group -Q-(CH₂)ₙ-Z, Q denoting an oxygen or sulphur atom, n the number 0, 1 or 2 and Z an alkyl, aryl or alkylaryl radical, substituted if desired.

2. Compounds according to Claim 1, in which m is equal to 2.

3. Compounds according to Claim 1 or 2, in which A is an ammonium group chosen from those of formula: 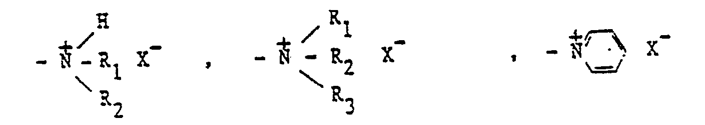 and 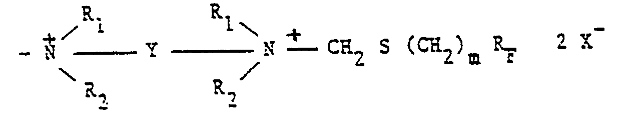 in which each of the symbols Rᵢ, R₂ and Rs, which are identical or different, denotes an alkyl, phenyl or benzyl radical, substituted if desired, Y denotes an alkylene bridge containing 2 to 6 carbon atoms interrupted, if desired, by an oxygen atom, and X-denotes a monovalent anion or its equivalent.

4. Compounds according to Claim 3, in which at least one of the radicals Ri, R₂ and R₃ is substituted by a halogen atom, a hydroxyl or nitrile group, or an ester, acid, sulphonate, sulphate or carboxylate functional group.

5. Compounds according to Claim 1 or 2, in which A is a phosphonium group: 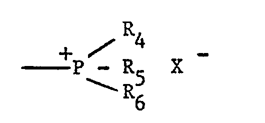 where each of the symbols R₄, R₅ and Rs, which are identical or different, denotes an alkyl or aryl radical and X- denotes a monovalent anion or its equivalent.

6. Compounds according to Claim 1 or 2, in which A is a group -Q-(CH₂)n-Z whose alkyl, aryl or alkylaryl radical Z carries at least one ionic or nonionic hydrophilic substitutent.

7. Compounds according to Claim 1 or 2, in which A is a group -Q-(CH₂)n-Z whose alkyl, aryl or alkylaryl radical Z is not substituted or is substituted by one or several nonhydrophilic substituents.

8. Process for the preparation of an alcohol of formula: 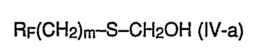 according to Claim 1, characterized in that trioxymethylene is reacted with a polyfluoroalkanethiol R_{F}-(CH₂)m-SH, in a proportion of three moles of thiol per one mole of trioxymethylene.

9. Process according to Claim 8, in which the operation is performed in the region of 0_{°}C under an inert gas atmosphere and in the absence of solvent.

10. Process for the preparation of a polyfluoroalkylthiomethyl halide of formula: 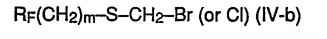 according to Claim 1, characterized in that a halogenating agent is reacted with the corresponding alcohol of formula:

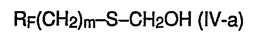

11. Process according to Claim 10, in which the operation is performed in the region of 0°C under an inert gas atmosphere and in an anhydrous ether.

12. Process for the preparation of the compounds according to Claim 1, in which A is an ammonium, phosphonium or -Q-(CH₂)ₙ-Z group, characterized in that a polyfluoroalkylthiomethyl halide of formula: 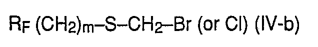 is reacted, respectively, with a secondary or tertiary amine, a phosphine, or an alcoholate or thiolate of formula: 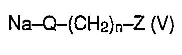 the symbols R_{F}, Q, m, n and Z having the same meanings as in Claim 1.

13. Use of the compounds according to one of Claims 3 to 6 as surface-active agents.

14. Use of the compounds according to Claim 7 as intermediates for the synthesis of surface-active agents.
